# EUROPÄISCHE PATENTANMELDUNG

(11) **EP 3 569 173 A1**
(43) Veröffentlichungstag der Anmeldung: **20.11.2019**
(21) Anmeldenummer: 18172947.6
(22) Anmeldetag: 17.05.2018
(51) Int. Cl.: A61B 18/14

(54) **KATHETER UND SYSTEM**

(71) Anmelder: VascoMed GmbH, 79589 Binzen (DE)
(72) Erfinder: Bitzer, Andreas, 8032 Zürich (CH)
(74) Vertreter: Engelhardt, Björn

(57) **Zusammenfassung**

Die Offenbarung betrifft einen Katheter mit einem ersten Sensor (2), der in einem ersten Abschnitt (1) des Katheters angeordnet ist, einem ersten beweglichen Bereich (8), der ein proximale Ende (4) des ersten Abschnitts (1) mit einem distalen Ende (6) eines zweiten Abschnitts (5) verbindet, derart, dass der erste Abschnitt (1) relativ zu dem zweiten Abschnitt (5) bewegbar ist, einem zweiten beweglichen Bereich (12), der ein proximales Ende (7) des zweiten Abschnitts (5) mit einem distalen Ende (11) eines dritten Abschnitts (9) verbindet, derart, dass der zweite Abschnitt (5) relativ zu dem dritten Abschnitt (9) bewegbar ist, und einem Referenzsensor (10), der in dem dritten Abschnitt (9) angeordnet ist. Eine Relativbewegung des ersten Sensors (2) zu dem Referenzsensor (10) ist auswertbar, um eine auf den ersten Abschnitt (1) einwirkende Kraft zu bestimmen. Weiterhin sind ein System mit einem Katheter und einer Auswerteeinheit offenbart.

## Beschreibung

Die Offenbarung betrifft einen Katheter sowie ein System mit einem Katheter und einer Auswerteeinheit.

### Hintergrund

Bei einer Ablation im Herzen (oder einem anderen Organ) ist es vorteilhaft, die Kraft zu bestimmen, mit welcher die Spitze eines Ablationskatheters auf das Gewebe drückt. Falls die Kraft zu gering ist, kann der Kontakt der Spitze zum Gewebe zu gering sein, was zu einer unzureichenden Läsion führen kann. Andererseits besteht bei zu hohem Druck auf das Gewebe die Gefahr einer Perforation.

Im Stand der Technik sind verschiedene Möglichkeiten zum Bestimmen der Kraft bekannt.

Das Dokument US 2017/0143416 A1 offenbart einen Katheter, der eingerichtet ist, eine Kontaktkraft zu bestimmen. In einem distalen Bereich des Katheters ist ein Federelement angeordnet. In dem Federelement sind mehrere Sensoren angeordnet, mit denen eine Verformung des Federelements bei einer Krafteinwirkung auf den Katheter bestimmt wird, um hieraus die Kraft zu ermitteln.

Eine ähnliche Technologie ist in den Dokumenten US 2017/0290617 A1 und US 2017/0172509 A1 offenbart.

Das Dokument US 2009/0093806 A1 offenbart die Verwendung von magnetischen Sensoren in einem beweglichen Bereich der Katheterspitze, um die Kraft zu bestimmen.

Das Dokument EP 2 711 676 A2 offenbart einen Katheter mit einem faseroptischen Kraftsensor.

Den bekannten Systemen ist gemeinsam, dass sie eine hohe Komplexität aufweisen.

### Zusammenfassung

Aufgabe ist es, verbesserte Technologien für die Kraftmessung bei Ablationen zur Verfügung zu stellen.

Es sind ein Katheter nach Anspruch 1 und ein System nach Anspruch 11 offenbart. Weitere Ausführungsformen sind Gegenstand von abhängigen Ansprüchen.

Nach einem Aspekt ist ein Katheter bereitgestellt. Der Katheter hat einen ersten Sensor, der in einem ersten Abschnitt des Katheters angeordnet ist, wobei der erste Abschnitt ein distales Ende und ein proximales Ende hat. Weiterhin weist der Katheter einen ersten beweglichen Bereich auf, der das proximale Ende des ersten Abschnitts mit einem distalen Ende eines zweiten Abschnitts verbindet, derart, dass der erste Abschnitt relativ zu dem zweiten Abschnitt bewegbar ist. Der Katheter hat des Weiteren einen zweiten beweglichen Bereich, der ein proximales Ende des zweiten Abschnitts mit einem distalen Ende eines dritten Abschnitts verbindet, derart, dass der zweite Abschnitt relativ zu dem dritten Abschnitt bewegbar ist. Ein Referenzsensor ist in dem dritten Abschnitt angeordnet. Eine Relativbewegung des ersten Sensors zu dem Referenzsensor ist auswertbar, um eine auf den ersten Abschnitt einwirkende Kraft zu bestimmen.

In dem zweiten Abschnitt kann ein zweiter Sensor angeordnet sein, wobei eine Relativbewegung des ersten Sensors zu dem Referenzsensor und/oder eine Relativbewegung des ersten Sensors zu dem zweiten Sensor und/oder eine Relativbewegung des zweiten Sensors zu dem Referenzsensor auswertbar ist, um eine auf den ersten Abschnitt einwirkende Kraft zu bestimmen.

Das distale Ende des ersten Abschnitts kann die Katheterspitze bilden. Der Katheter kann ein Ablationskatheter sein. An dem ersten Abschnitt des Katheters kann eine Elektrode angeordnet sein, beispielsweise eine Ablationselektrode. Die Ablationselektrode kann an der Katheterspitze angeordnet sein. Weitere Elektroden können an dem ersten Abschnitt, an dem zweiten Abschnitt und/oder an dem dritten Abschnitt angeordnet sein. Die weiteren Elektroden können ringförmig sein und beispielsweise als Messelektroden ausgeführt sein.

Im Sinne der vorliegenden Offenbarung bedeutet "proximal" eine geringere Entfernung einer solchermaßen gekennzeichneten Komponente oder eines solchermaßen gekennzeichneten Ortes entlang des Verlaufs des Katheters, im Vergleich zu einer als "distal" bezeichneten Komponente bzw. eines als "distal" bezeichneten Ortes, die bzw. der eine größere Entfernung zu dem Bediener entlang des Verlaufs des Katheters aufweist.

Der erste Abschnitt und der zweite Abschnitt können beweglich sein und beispielsweise aus einem biegsamen Material geformt sein. Der dritte Abschnitt kann unbeweglich ausgeführt sein. Der dritte Abschnitt kann aus einem festen (nicht biegsamen) Material gebildet sein und/oder von einem festen (nicht biegsamen) Material umgeben sein, so dass eine Bewegung und/oder Verformung des dritten Abschnitts verhindert wird.

Der Katheter kann als gespülter Katheter gebildet sein. In der Elektrode können mehrere Spülöffnungen gebildet sein, die mit einer innerhalb des Katheters angeordneten Spülzuleitung verbunden sind.

Nach einem weiteren Aspekt ist ein System mit einem Katheter und einer Auswerteeinheit bereitgestellt. Die Auswerteeinheit ist mit dem Katheter gekoppelt und eingerichtet, anhand einer Relativbewegung des ersten Sensors zu dem Referenzsensor eine auf den ersten Abschnitt des Katheters einwirkende Kraft zu bestimmen.

Die Auswerteeinheit kann eingerichtet sein, eine Änderung des Abstands zwischen dem ersten Sensor und dem Referenzsensor auszuwerten, um die Kraft zu bestimmen und/oder eine Änderung des Winkels zwischen dem ersten Sensor und dem Referenzsensor auszuwerten, um die Kraft zu bestimmen.

Falls der Katheter den zweiten Sensor in dem zweiten Abschnitt aufweist, kann die Auswerteeinheit eingerichtet sein, eine Relativbewegung des ersten Sensors zu dem Referenzsensor und/oder eine Relativbewegung des ersten Sensors zu dem zweiten Sensor und/oder eine Relativbewegung des zweiten Sensors zu dem Referenzsensor auszuwerten, um eine auf den ersten Abschnitt einwirkende Kraft zu bestimmen. Die Auswerteeinheit kann eingerichtet sein, eine Änderung des Abstands zwischen dem ersten Sensor und dem Referenzsensor und/oder eine Änderung des Abstands zwischen dem ersten Sensor und dem zweiten Sensor und/oder eine Änderung des Abstands zwischen dem zweiten Sensor und dem Referenzsensor auszuwerten, um die Kraft zu bestimmen. Ergänzend oder alternativ kann die Auswerteeinheit eingerichtet sein, eine Änderung des Winkels zwischen dem ersten Sensor und dem Referenzsensor und/oder eine Änderung des Winkels zwischen dem ersten Sensor und dem zweiten Sensor und/oder eine Änderung des Winkels zwischen dem zweiten Sensor und dem Referenzsensor auszuwerten, um die Kraft zu bestimmen. Eine winkelaufgelöste Bestimmung der Kraft kann genauer sein als eine positionsaufgelöste Bestimmung (anhand des Abstands) der Kraft.

Die Auswerteeinheit kann einen Prozessor und einen Speicher aufweisen und beispielsweise als Computer, Server oder Tablet ausgeführt sein. Die Auswerteeinheit kann mit einer Anzeigevorrichtung (z. B. einem Monitor) gekoppelt sein, um die Kraft anzuzeigen.

Der erste Sensor, der Referenzsensor und der zweite Sensor können magnetische Sensoren sein, beispielsweise Spulen, die mit einem externen Magnetfeld angeregt werden. Das System kann in diesem Fall eine Vorrichtung zum Erzeugen eines externen Magnetfelds aufweisen. Das externe Magnetfeld kann beispielsweise ein magnetisches Wechselfeld sein, bei dem Amplitude und/oder Polung des Feldes zeitlich veränderlich sind.

Die Sensoren können eine Winkelauflösung von 0,2° haben. Der relative Winkelfehler zwischen zwei Sensoren beträgt in diesem Fall 0,4°. Für eine Abstandsmessung kann die absolute Genauigkeit der Sensoren 1 mm betragen, was zu einer relativen Genauigkeit von 2 mm führt. Hiermit kann eine ausreichende Genauigkeit für die geplanten Anwendungsfälle (z. B. geforderte Auflösung ΔF = 0,025 N (entspricht 2,5 Gramm)) erzielt werden. Bei einer Krafteinwirkung kommt es üblicherweise nur zu geringfügigen Verformungen des Katheters, also geringen Auslenkungen. Die Auswertung der Winkel mittels drei Sensoren kann daher vorteilhaft sein (gegenüber zwei Sensoren).

Der erste bewegliche Bereich kann als ein erstes Gelenk zwischen dem ersten Abschnitt und dem zweiten Abschnitt ausgebildet sein. Der zweite bewegliche Bereich kann als ein zweites Gelenk zwischen dem zweiten Abschnitt und dem dritten Abschnitt ausgebildet sein. Ein Gelenk ist eine bewegliche Verbindung zwischen zwei Teilen, beispielsweise mittels einer Drehbewegung oder einer Schubbewegung.

Das erste Gelenk kann als eine erste Einkerbung zwischen dem proximalen Ende des ersten Abschnitts und dem distalen Ende des zweiten Abschnitts gebildet sein. Das zweite Gelenk kann als eine zweite Einkerbung zwischen dem proximalen Ende des zweiten Abschnitts und dem distalen Ende des dritten Abschnitts gebildet sein. Der erste Abschnitt kann einteilig mit dem zweiten Abschnitt gebildet sein. Die erste Einkerbung (Aussparung) zwischen dem ersten Abschnitt und dem zweiten Abschnitt führt dazu, dass der erste Abschnitt relativ zu dem zweiten Abschnitt bewegt werden kann. Beispielsweise kann der erste Abschnitt gegenüber dem zweiten Abschnitt geknickt werden. Dies gilt analog für die zweite Einkerbung.

Die erste Einkerbung kann eine einseitige Einkerbung oder eine zentrische Einkerbung sein. Die zweite Einkerbung kann eine einseitige Einkerbung oder eine zentrische Einkerbung sein. Eine einseitige Einkerbung entsteht, wenn auf einer Seite eine Aussparung in dem Material gebildet wird. In diesem Fall bleibt an einer anderen Seite ein Teil des Materials übrig, das den ersten Abschnitt mit dem zweiten Abschnitt (oder den zweiten Abschnitt mit dem dritten Abschnitt) verbindet. Eine zentrische Einkerbung entsteht, wenn von zwei Seiten gleich viel Material entfernt wird, so dass in der Mitte ein Teil des Materials verbleibt, das die Abschnitte verbindet.

In einer Ausführungsform sind die erste Einkerbung und die zweite Einkerbung jeweils als einseitige Einkerbung gebildet, wobei die Einkerbungen auf den gleichen Seiten gebildet sein können. In einer anderen Ausführungsform ist die erste Einkerbung als einseitige Einkerbung gebildet und die zweite Einkerbung ist als zentrische Einkerbung gebildet.

Mit den Einkerbungen wird ein anisotropisches Ansprechverhalten auf einwirkende Kräfte erreicht.

Es kann vorgesehen sein, dass der zweite bewegliche Bereich mit zwei hintereinander angeordneten Einkerbungen zwischen dem proximalen Ende des zweiten Abschnitts und dem distalen Ende des dritten Abschnitts gebildet ist, wobei eine näher am proximalen Ende des zweiten Abschnitts angeordnete Einkerbung als zentrische Einkerbung gebildet ist und wobei eine näher am distalen Ende des dritten Abschnitts angeordnete Einkerbung als eine einseitige Einkerbung gebildet ist.

In einer weiteren Ausführungsform kann der erste bewegliche Bereich als ein Federelement ausgebildet sein, wobei das Federelement das proximale Ende des ersten Abschnitts mit dem distalen Ende des zweiten Abschnitts verbindet. Der zweite bewegliche Bereich kann als Gelenk zwischen dem zweiten Abschnitt und dem dritten Abschnitt ausgebildet sein. Für das Gelenk gelten die obigen Ausführungen zum zweiten Gelenk analog.

Es kann vorgesehen sein, dass der erste Abschnitt, der zweite Abschnitt und der dritte Abschnitt einteilig gebildet sind und einen S-förmigen Verlauf aufweisen, wobei der erste bewegliche Bereich in einem ersten Bogen des S-förmigen Verlaufs gebildet ist und wobei der zweite bewegliche Bereich in einem zweiten Bogen des S-förmigen Verlaufs gebildet ist.

### Beschreibung von Ausführungsbeispielen

Im Folgenden werden beispielhafte Ausführungsformen unter Bezugnahme auf Figuren näher erläutert. Es zeigen:
- Fig. 1: eine schematische Ansicht einer Ausführungsform eines Katheters mit zwei Sensoren,
- Fig. 2: eine schematische Ansicht einer Ausführungsform eines Katheters mit drei Sensoren,
- Fig. 3: eine schematische Ansicht einer weiteren Ausführungsform eines Katheters mit zwei Sensoren,
- Fig. 4: eine Verformung des Katheters nach Fig. 3 bei Einwirkung einer Kraft entlang der z-Richtung,
- Fig. 5: eine Verformung des Katheters nach Fig. 3 bei Einwirkung einer Kraft entlang der x-Richtung,
- Fig. 6: eine schematische Ansicht einer weiteren Ausführungsform eines Katheters mit drei Sensoren,
- Fig. 7: eine Verformung des Katheters nach Fig. 6 bei Einwirkung einer Kraft entlang der x-Richtung,
- Fig. 8: eine Verformung des Katheters nach Fig. 6 bei Einwirkung einer Kraft entlang der z-Richtung,
- Fig. 9: eine Verformung des Katheters nach Fig. 6 bei Einwirkung einer Kraft entlang der y-Richtung,
- Fig. 10: eine schematische Ansicht einer anderen Ausführungsform eines Katheters mit drei Sensoren,
- Fig. 11: schematische Ansichten von Verformungen des Katheters nach Fig. 2 bei Einwirkung von Kräften,
- Fig. 12: eine schematische Ansicht einer noch weiteren Ausführungsform eines Katheters mit drei Sensoren,
- Fig. 13: schematische Ansichten eines S-förmigen Katheters und
- Fig. 14: ein Blockdiagramm eines Systems.

Für gleiche Komponenten werden im Folgenden gleiche Bezugszeichen verwendet.

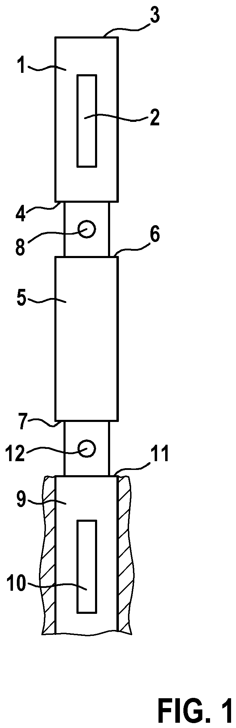

Fig. 1 zeigt eine schematische Ansicht eines distalen Bereichs eines Katheters. Der Katheter umfasst einen ersten Abschnitt 1 mit einem distalen Ende 3 und einem proximalen Ende 4. Das distale Ende 3 des ersten Abschnitts 1 bildet die Spitze des Katheters. In dem ersten Abschnitt 1 ist ein Sensor 2 angeordnet. Der Sensor 2 kann ein magnetischer Sensor sein, beispielsweise eine Spule.

Unterhalb des ersten Abschnitts 1 befindet sich ein zweiter Abschnitt 5. Zwischen dem proximalen Ende 4 des ersten Abschnitts 1 und einem distalen Ende 6 des zweiten Abschnitts 5 ist ein erster beweglicher Bereich 8 gebildet. Hierdurch ist der erste Abschnitt 1 relativ zu dem zweiten Abschnitt 5 bewegbar, beispielsweise bei Einwirkung einer Kraft auf den ersten Abschnitt 1.

Der Katheter umfasst des Weiteren einen dritten Abschnitt 9. Zwischen einem proximalen Ende 7 des zweiten Abschnitts 5 und einem distalen Ende 11 des dritten Abschnitts 9 ist ein zweiter beweglicher Bereich 12 gebildet. Hierdurch ist der zweite Abschnitt 5 relativ zu dem dritten Abschnitt 9 bewegbar. In dem dritten Abschnitt 9 ist ein Referenzsensor 10 angeordnet. Der Referenzsensor 10 kann ebenfalls ein magnetischer Sensor sein, beispielsweise eine Spule.

Der dritte Abschnitt 9 ist steif. Wenn eine Kraft auf den ersten Abschnitt 1 einwirkt, werden der erste Abschnitt 1 und unter Umständen auch der zweite Abschnitt 5 relativ zu dem dritten (fixierten) Abschnitt 9 bewegt. Hierdurch ändert sich die Position und/oder die Ausrichtung des ersten Sensors 2 zu dem Referenzsensor 10. Anhand der Änderung der Position und/oder der Ausrichtung kann die Kraft bestimmt werden.

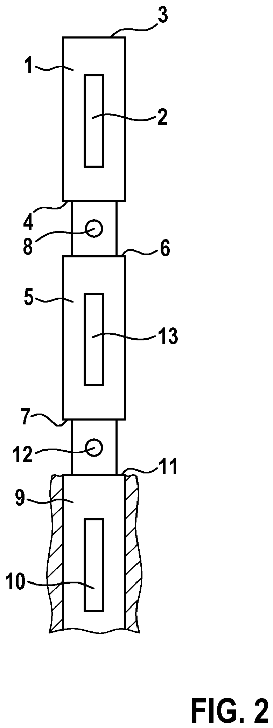

In Fig. 2 ist eine Ausführungsform des Katheters gezeigt, wobei in dem zweiten Abschnitt 5 ein zweiter Sensor 13 angeordnet ist. Der zweite Sensor 13 kann ebenfalls ein magnetischer Sensor sein, z. B. eine Spule. Der erste Sensor 2, der zweite Sensor 13 und der Referenzsensor 10 können baugleiche Sensoren sein, z. B. baugleiche Spulen. Als Sensoren können 5D-Navigations-Magenetfeldsensoren verwendet werden, wie sie von der Firma NDI (System Aurora) erhältlich sind.

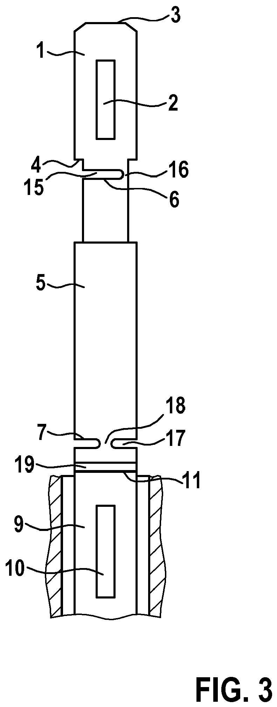

Fig. 3 zeigt eine Ausführungsform, wobei der erste bewegliche Bereich und der zweite bewegliche Bereich mit Gelenken realisiert sind. Zwischen dem proximalen Ende 4 des ersten Abschnitts 1 und dem distalen Ende 6 des zweiten Abschnitts 5 ist eine erste seitliche Einkerbung 15 gebildet. Ein Teil des Materials ist hier entfernt, wobei ein erstes seitliches Wandelement 16 verbleibt, das den ersten Abschnitt 1 mit dem zweiten Abschnitt 5 verbindet. Die Kombination aus der ersten seitlichen Einkerbung 15 und dem ersten seitlichen Wandelement 16 bildet ein Gelenk. Die erste seitliche Einkerbung 15 ermöglicht ein Verkippen des ersten Abschnitts 1 gegenüber dem zweiten Abschnitt 5, wenn eine Kraft auf den ersten Abschnitt 1 wirkt.

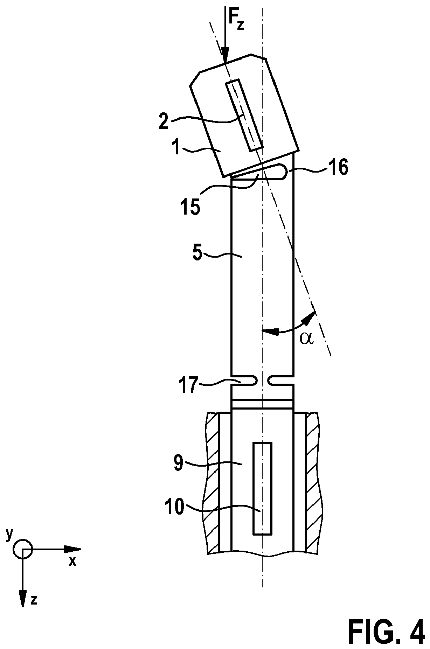

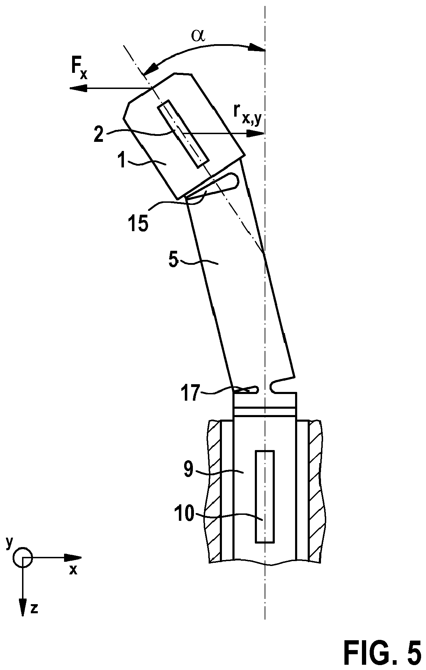

Zwischen dem zweiten Abschnitt 5 und dem dritten Abschnitt 9 ist ein weiteres Gelenk gebildet. An das proximale Ende 7 des zweiten Abschnitts 5 grenzt eine zentrische Einkerbung 17 mit einem zentrischen Wandelement 18. Des Weiteren ist eine zweite seitliche Einkerbung 19 gebildet, welche an das distale Ende 11 des dritten Abschnitts 9 grenzt. Zu der zweiten seitlichen Einkerbung 19 gehört ein zweites seitliches Wandelement (analog wie bei der ersten seitlichen Einkerbung). Das zweite seitliche Wandelement ist bei der gezeigten Perspektive hinter der zweiten seitlichen Einkerbung 19 gebildet (also auf der Rückseite des Katheters) und daher nicht dargestellt. Durch diese Konfiguration der Einkerbungen weist der Katheter ein anisotropes Ansprechverhalten auf eine Kraft auf. Die Kraftmessung wird im Folgenden mit Bezug auf die Fig. 4 und 5 näher erläutert.

In Fig. 4 ist die Wirkung einer Kraft in z-Richtung (entlang der Achse des Katheters) auf die Spitze des Katheters nach Fig. 3 dargestellt. Der erste Abschnitt 1 wird gegenüber dem zweiten Abschnitt 5 gekippt. Die Verkippung erfolgt auf der Seite der ersten seitlichen Einkerbung 15. Durch die Verkippung wird ein Winkel α zwischen dem ersten Sensor 1 und dem Referenzsensor 10 gebildet. Des Weiteren kommt es zu einer (recht geringfügigen) Änderung des Abstands *r_{xy}* zwischen dem ersten Sensor 1 und dem Referenzsensor 10. Anhand der Positionen des ersten Sensors 2 und des Referenzsensors 10 können der Winkel α und der Abstand *r_{xy}* bestimmt werden. Hieraus kann anschließend die Kraft berechnet werden.

Fig. 5 zeigt den Katheter von Fig. 3, wobei eine Kraft in x-Richtung (von der Seite) auf den ersten Abschnitt 1 des Katheters wirkt. Der Katheter wird dadurch gekippt und es ändern sich sowohl der Winkel α sowie der Abstand *r_{xy}* zwischen dem ersten Sensor 2 und dem Referenzsensor 10. Aus diesen Größen kann wiederum die Kraft bestimmt werden.

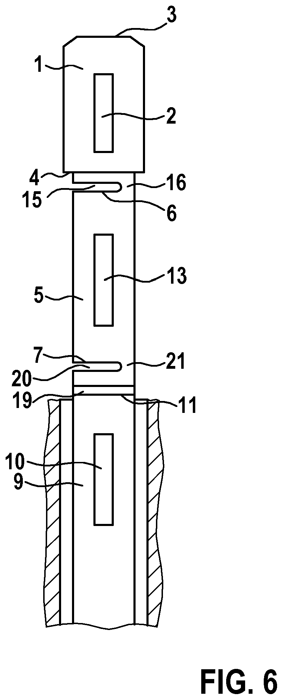

Fig. 6 zeigt einen Katheter mit drei Sensoren. In dem zweiten Abschnitt 5 ist der zweite Sensor 13 angeordnet. Zwischen dem ersten Abschnitt 1 und dem zweiten Abschnitt 5 ist die erste seitliche Einkerbung 15 mit dem ersten seitlichen Wandelement 16 gebildet. Am distalen Ende 11 des dritten Abschnitts 9 ist die zweite seitliche Einkerbung 19 gebildet (das zweite seitliche Wandelement ist hier wiederum nicht dargestellt, weil es "hinter" der zweiten seitlichen Einkerbung liegt). Am proximalen Ende 7 des zweiten Abschnitts 5 ist eine dritte seitliche Einkerbung 20 mit einem dritten seitlichen Wandelement 21 gebildet. Die dritte seitliche Einkerbung 20 liegt auf der gleichen Seite wie die erste seitliche Einkerbung 15.

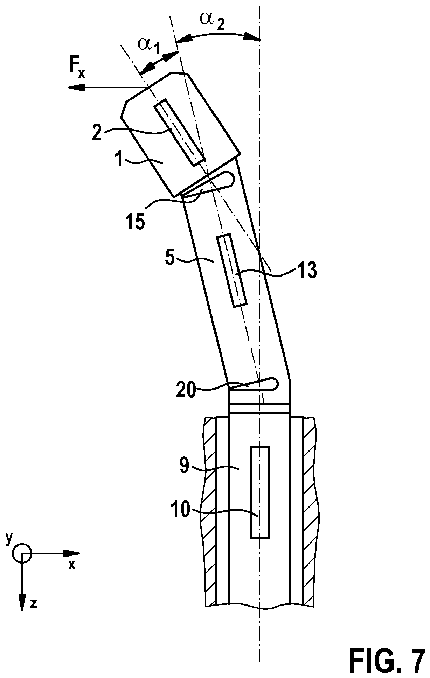

Die Wirkung einer Kraft in x-Richtung auf den Katheter aus Fig. 6 ist in Fig. 7 dargestellt. Zwischen dem ersten Sensor 2 und dem zweiten Sensor 13 ist ein erster Winkel *α*₁ gebildet. Zwischen dem zweiten Sensor 13 und dem Referenzsensor 10 ist ein zweiter Winkel *α*₂ gebildet.

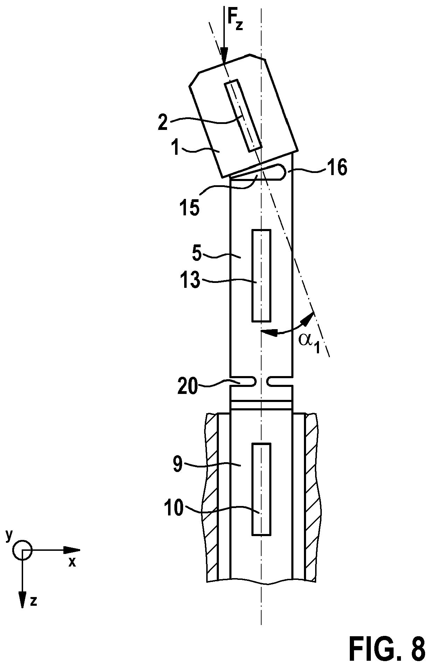

Fig. 8 zeigt die Wirkung einer Kraft in z-Richtung auf den Katheter gemäß Fig. 6. Das Abkippen des ersten Abschnitts 1 an der ersten seitlichen Einkerbung 15 führt zu einem ersten Winkel *α*₁ zwischen dem ersten Sensor 2 und dem zweiten Sensor 13.

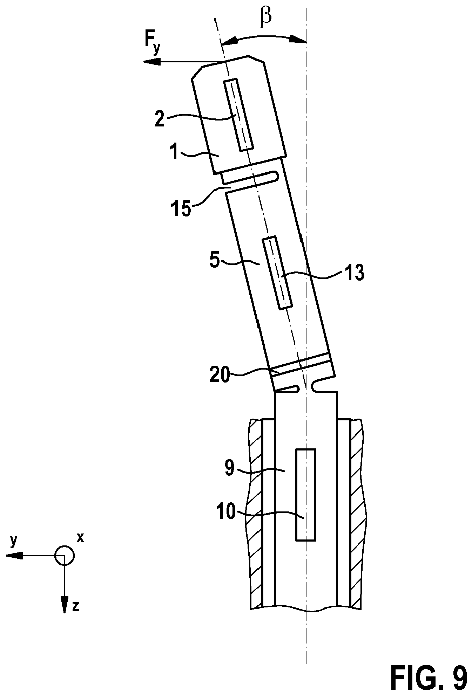

Fig. 9 zeigt die Wirkung einer Kraft in y-Richtung auf den Katheter nach Fig. 6. Hierbei bewegt sich der erste Abschnitt 1 nicht relativ zum zweiten Abschnitt 5, weil das erste seitliche Wandelement 16 eine Versteifung bildet und die Bewegung verhindert. Der erste Abschnitt 1 und der zweite Abschnitt 5 bleiben in einer geraden Linie zueinander und werden gemeinsam gegenüber dem dritten Abschnitt 9 gekippt. Dies führt zu einem Winkel β.

Eine Auswertung der Winkel und ggf. der Abstände der Sensoren zueinander (insbesondere des Abstands zwischen dem ersten Sensor und dem Referenzsensor sowie des Abstands des zweiten Sensors zu dem Referenzsensor) ermöglicht die Bestimmung der Kraft.

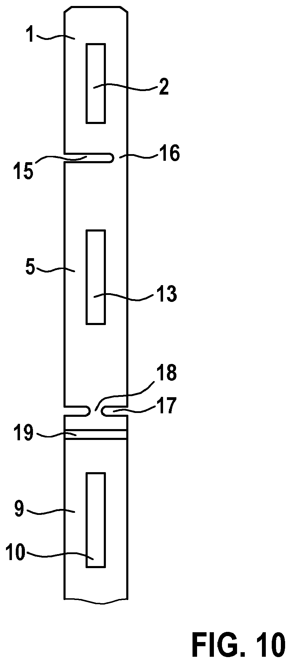

In Fig. 10 ist eine andere Ausführungsform eines Katheters mit drei Sensoren gezeigt. Zwischen dem ersten Abschnitt 1 und dem zweiten Abschnitt 5 ist die erste seitliche Einkerbung 15 mit dem ersten seitlichen Wandelement 16 gebildet. Am distalen Ende des dritten Abschnitts ist die zweite seitliche Einkerbung 19 gebildet. Am proximalen Ende des zweiten Abschnitts 5 ist die zentrische Einkerbung 17 mit dem zentrischen Wandelement 18 gebildet.

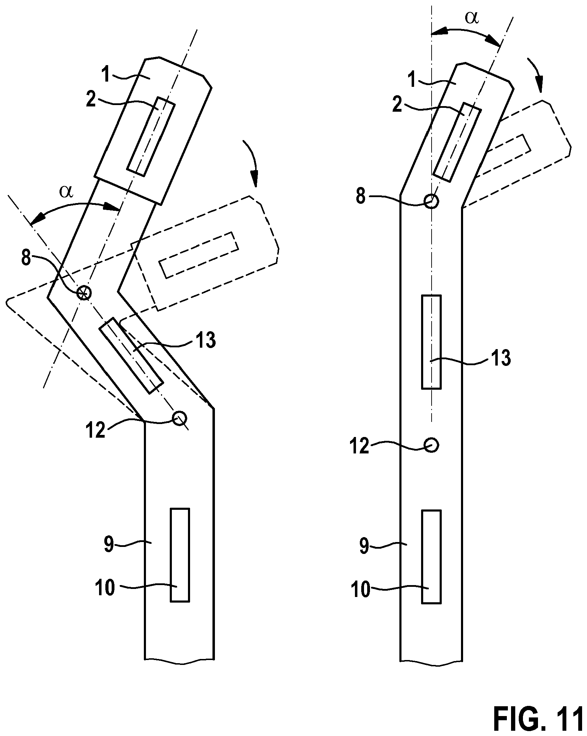

Verschiedene Konfigurationen und Bewegungen der einzelnen Abschnitte des Katheters sind schematisch in Fig. 11 gezeigt.

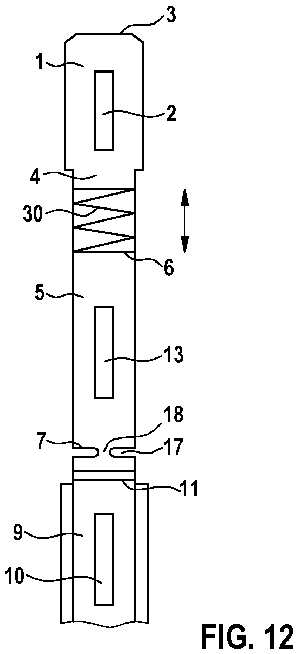

Fig. 12 zeigt eine weitere Ausführungsform des Katheters mit einem Federelement 30 zwischen dem ersten Abschnitt 1 und dem zweiten Abschnitt 5. Mit dieser "Teleskop"-Anordnung können die Winkel α und β sowie der Abstand d zwischen dem ersten Sensor 2 und dem zweiten Sensor 13 bestimmt werden, um hieraus die Kraft zu bestimmen.

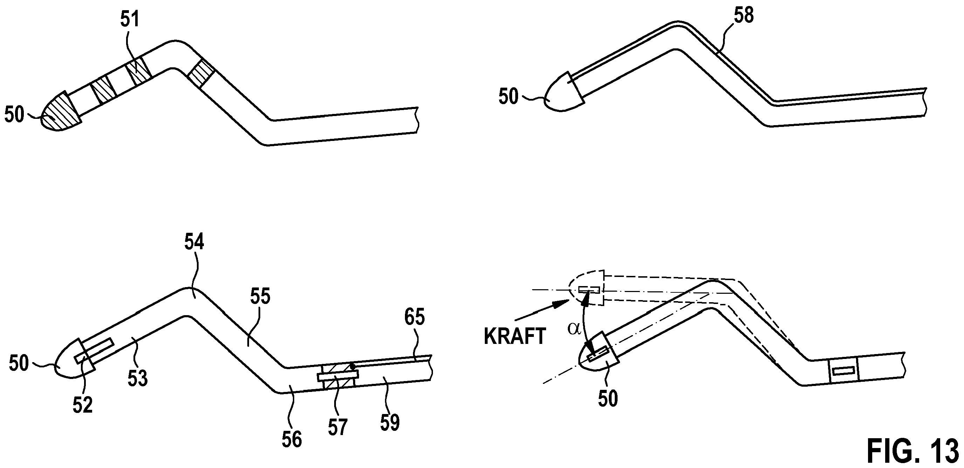

Fig. 13 zeigt eine S-förmige Ausführungsform des Katheters. Oben links von Fig. 13 ist der Katheter mit einer Ablationselektrode 50 und mehreren ringförmigen Messelektroden 51 gezeigt.

In einem ersten Abschnitt 53 ist ein erster Sensor 52 (auch Tip-Sensor genannt) angeordnet. Der erste Abschnitt 53 ist mit einem ersten beweglichen Bereich 54 mit einem zweiten Abschnitt 55 verbunden. Der zweite Abschnitt 55 ist mit einem zweiten beweglichen Bereich 56 mit einem dritten Abschnitt 59 verbunden. In dem dritten Abschnitt 59 ist ein Referenzsensor 57 angeordnet. Ein distales Ende des dritten Abschnitts ist mit einem Zugdraht 65 verbunden. Der Referenzsensor 57 ist an der Zugdrahtbefestigung angeordnet. Der Zugdraht 65 ermöglicht eine Deflektion des Katheters.

Ein flexibler Draht 58 ist innerhalb des Katheters angeordnet und bestimmt die S-Form des Katheters (oben rechts von Fig. 13).

Beim Einwirken einer Kraft auf die Spitze des Katheters verformt sich der Katheter (vgl. unten rechts von Fig. 13). Dies führt zu einer Änderung des Winkels α zwischen dem ersten Sensor 52 und dem Referenzsensor 57. Auch die Position des erstens Sensors 52 ändert sich bei der Krafteinwirkung.

Zur Ermittlung des Kraftvektors kann eine Matrix verwendet werden. Die Matrix bildet die Messwerte, welche sich aus der Differenz zwischen Tip-Sensor und dem Referenzsensor ergeben (Änderung der Position und Änderung der Winkel), auf einen dreidimensionalen (3D) Kraftvektor ab. Es ist auch denkbar die Abbildung numerisch und diskret durchzuführen.

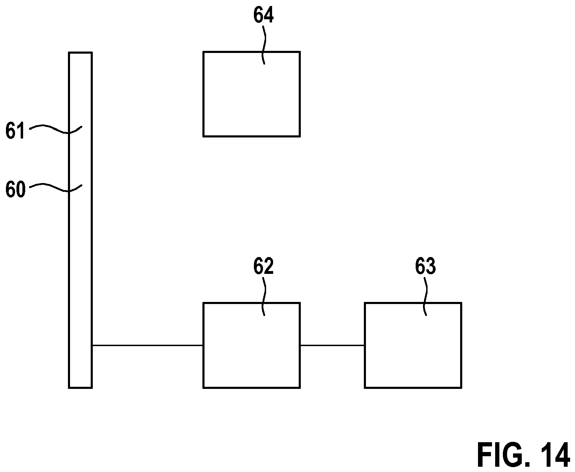

In Fig. 14 ist schematisch ein System mit einem Katheter 60, einer Auswerteeinheit 62 und einer Anzeigeeinrichtung 63 dargestellt. Ein distaler Bereich 61 des Katheters 60 ist nach einer der hier offenbarten Ausführungsformen gebildet, weist also zwei oder drei Sensoren (z. B. magnetische Sensoren) sowie bewegliche Bereiche auf Die Auswerteeinheit 62 ist mit dem Katheter 60 und der Anzeigeeinrichtung 63 gekoppelt. Des Weiteren ist eine Vorrichtung zum Erzeugen eines Magnetfelds vorgesehen. Das Magnetfeld wirkt auf den distalen Bereich 61 des Katheters 60, so dass die Relativbewegung der Sensoren bestimmt werden kann.

Der Katheter gemäß der vorliegenden Offenbarung kann die folgenden Vorteile haben:
- Die sehr genaue Magnetfeld-Navigations-Sensorik lässt sich zur Kraftmessung verwenden.
- Es ist keine zusätzliche Messtechnik notwendig, z. B. keine optische Fasermesstechnik.
- Der Katheter hat einen flexiblen und robusten distalen Bereich (Tip-Bereich), so dass die Gefahr einer Beschädigung bei Schleusen-Transit oder beim Einführen in die Schleuse verringert ist.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste:

- 1: erster Abschnitt
- 2: erster Sensor
- 3: distales Ende des ersten Abschnitts
- 4: proximales Ende des ersten Abschnitts
- 5: zweiter Abschnitt
- 6: distales Ende des zweiten Abschnitts
- 7: proximales Ende des zweiten Abschnitts
- 8: erster beweglicher Bereich
- 9: dritter Abschnitt
- 10: Referenzsensor
- 11: distales Ende des dritten Bereichs
- 12: zweiter beweglicher Bereich
- 13: zweiter Sensor
- 15: erste seitliche Einkerbung
- 16: erstes seitliches Wandelement
- 17: zentrische Einkerbung
- 18: zentrisches Wandelement
- 19: zweite seitliche Einkerbung
- 20: dritte seitliche Einkerbung
- 21: drittes seitliches Wandelement
- 30: Federelement
- 50: Ablationselektrode
- 51: Messelektrode
- 52: erster Sensor
- 53: erster Abschnitt
- 54: erster beweglicher Bereich
- 55: zweiter Abschnitt
- 56: zweiter beweglicher Bereich
- 57: Referenzsensor
- 58: Draht
- 59: dritter Abschnitt
- 60: Katheter
- 61: distaler Bereich des Katheters
- 62: Auswerteeinheit
- 63: Anzeigeeinrichtung
- 64: Vorrichtung zum Erzeugen eines Magnetfelds
- 65: Zugdraht

## Patentansprüche

1. Katheter mit
- einem ersten Sensor (2), der in einem ersten Abschnitt (1) des Katheters angeordnet ist, wobei der erste Abschnitt (1) ein distales Ende (3) und ein proximales Ende (4) hat,
- einem ersten beweglichen Bereich (8), der das proximale Ende (4) des ersten Abschnitts (1) mit einem distalen Ende (6) eines zweiten Abschnitts (5) verbindet, derart, dass der erste Abschnitt (1) relativ zu dem zweiten Abschnitt (5) bewegbar ist,
- einem zweiten beweglichen Bereich (12), der ein proximales Ende (7) des zweiten Abschnitts (5) mit einem distalen Ende (11) eines dritten Abschnitts (9) verbindet, derart, dass der zweite Abschnitt (5) relativ zu dem dritten Abschnitt (9) bewegbar ist, und
- einem Referenzsensor (10), der in dem dritten Abschnitt (9) angeordnet ist,
wobei eine Relativbewegung des ersten Sensors (2) zu dem Referenzsensor (10) auswertbar ist, um eine auf den ersten Abschnitt (1) einwirkende Kraft zu bestimmen.

2. Katheter nach Anspruch 1, wobei in dem zweiten Abschnitt (5) ein zweiter Sensor (13) angeordnet ist, wobei eine Relativbewegung des ersten Sensors (2) zu dem Referenzsensor (10) und/oder eine Relativbewegung des ersten Sensors (2) zu dem zweiten Sensor (13) und/oder eine Relativbewegung des zweiten Sensors (13) zu dem Referenzsensor (10) auswertbar ist, um eine auf den ersten Abschnitt (1) einwirkende Kraft zu bestimmen.

3. Katheter nach Anspruch 1 oder 2, wobei der erste Sensor (2), der Referenzsensor (10) und ggf. der zweite Sensor (13) magnetische Sensoren sind.

4. Katheter nach einem der vorangehenden Ansprüche, wobei der erste bewegliche Bereich (8) als ein erstes Gelenk zwischen dem ersten Abschnitt (1) und dem zweiten Abschnitt (5) ausgebildet ist und/oder wobei der zweite bewegliche Bereich (12) als ein zweites Gelenk zwischen dem zweiten Abschnitt (5) und dem dritten Abschnitt (9) ausgebildet ist.

5. Katheter nach Anspruch 4, wobei das erste Gelenk als eine erste Einkerbung (15) zwischen dem proximalen Ende (4) des ersten Abschnitts (1) und dem distalen Ende (6) des zweiten Abschnitts (5) gebildet ist und/oder wobei das zweite Gelenk als eine zweite Einkerbung (17, 19, 20) zwischen dem proximalen Ende (7) des zweiten Abschnitts (5) und dem distalen Ende (11) des dritten Abschnitts (9) gebildet ist.

6. Katheter nach Anspruch 5, wobei die erste Einkerbung (15) eine einseitige Einkerbung oder eine zentrische Einkerbung ist.

7. Katheter nach Anspruch 5 oder 6, wobei die zweite Einkerbung (17, 19, 20) eine einseitige Einkerbung oder eine zentrische Einkerbung ist.

8. Katheter nach einem der vorangehenden Ansprüche, wobei der zweite bewegliche Bereich mit zwei hintereinander angeordneten Einkerbungen zwischen dem proximalen Ende (7) des zweiten Abschnitts (5) und dem distalen Ende (11) des dritten Abschnitts (9) gebildet ist, wobei eine näher am proximalen Ende (7) des zweiten Abschnitts (5) angeordnete Einkerbung als zentrische Einkerbung (17) gebildet ist und wobei eine näher am distalen Ende (11) des dritten Abschnitts (9) angeordnete Einkerbung als eine einseitige Einkerbung (20) gebildet ist.

9. Katheter nach einem der Ansprüche 1 bis 3, wobei der erste bewegliche Bereich (8) als ein Federelement (30) ausgebildet ist, wobei das Federelement (30) das proximale Ende (4) des ersten Abschnitts (1) mit dem distalen Ende (6) des zweiten Abschnitts (5) verbindet und wobei der zweite bewegliche Bereich (12) als Gelenk zwischen dem zweiten Abschnitt (5) und dem dritten Abschnitt (9) ausgebildet ist.

10. Katheter nach Anspruch 1, wobei der erste Abschnitt (53), der zweite Abschnitt (55) und der dritte Abschnitt (59) einteilig gebildet sind und einen S-förmigen Verlauf aufweisen, wobei der erste bewegliche Bereich (54) in einem ersten Bogen des S-förmigen Verlaufs gebildet ist und wobei der zweite bewegliche Bereich (56) in einem zweiten Bogen des S-förmigen Verlaufs gebildet ist.

11. System mit einem Katheter (60) nach einem der vorangehenden Ansprüche und einer Auswerteeinheit (62), die mit dem Katheter (60) gekoppelt ist, wobei die Auswerteeinheit (62) eingerichtet ist, anhand einer Relativbewegung des ersten Sensors zu dem Referenzsensor eine auf den ersten Abschnitt des Katheters (60) einwirkende Kraft zu bestimmen.

12. System nach Anspruch 11, wobei die Auswerteeinheit (62) eingerichtet ist, eine Änderung des Abstands zwischen dem ersten Sensor und dem Referenzsensor auszuwerten, um die Kraft zu bestimmen und/oder wobei die Auswerteeinheit (62) eingerichtet ist, eine Änderung des Winkels zwischen dem ersten Sensor und dem Referenzsensor auszuwerten, um die Kraft zu bestimmen.
